# EUROPÄISCHE PATENTSCHRIFT

(11) **EP 1 970 007 B1**
(45) Veröffentlichungstag und Bekanntmachung des Hinweises auf die Patenterteilung: **01.07.2009**
(21) Anmeldenummer: 07005231.1
(22) Anmeldetag: 14.03.2007
(51) Int. Cl.: A61B 5/15

(54) **Lanzettenvorrichtung**
Lancet device
Dispositif de lancettes

(43) Veröffentlichungstag der Anmeldung: 17.09.2008
(73) Patentinhaber: Roche Diagnostics GmbH, 68305 Mannheim (DE); F.HOFFMANN-LA ROCHE AG, 4070 Basel (CH)
(72) Erfinder: List, Hans, 64754 Hesseneck-Kailbach (DE)
(74) Vertreter: Jany, Peter

(56) Entgegenhaltungen:
- WO-A-20/07006399
- US-A- 5 147 375
- US-A- 6 080 172

## Beschreibung

Die Erfindung betrifft eine Lanzettenvorrichtung, mit der eine Lanzette entlang eines Einstichweges zum Erzeugen einer Einstichwunde in einer Hautoberfläche, insbesondere zum Gewinnen von Körperflüssigkeit für Diagnosezwecke, verschiebbar ist, umfassend einen Lanzettenantrieb, der ein Antriebsmittel zum Erzeugen einer Antriebskraft für eine Einstichbewegung der Lanzette entlang des Einstichweges in Richtung auf die Hautoberfläche umfasst, ein Rückhaltemittel zum Erzeugen einer magnetischen Haltekraft zum Halten der Lanzette in einer gespannten Position, und ein Auslösemittel, mit dem die Haltekraft soweit reduzierbar ist, dass die Lanzette unter Einwirkung der von dem Antriebsmittel erzeugten Antriebskraft in Richtung auf die Hautoberfläche beschleunigt wird.

Die Erfindung kann verwendet werden bei einem Verfahren zum Betreiben einer Lanzettenvorrichtung, mit der eine Lanzette entlang eines Einstichweges zum Erzeugen eine Einstichwunde in einer Hautoberfläche, insbesondere zum Gewinnen von Körperflüssigkeit für Diagnosezwecke, verschoben wird, umfassend einen Lanzettenantrieb, mit dem mit einem Antriebsmittel eine Antriebskraft für eine Einstichbewegung der Lanzette entlang des Einstichweges in Richtung auf die Hautoberfläche erzeugt wird, mit einem Rückhaltemittel eine magnetische Haltekraft zum Halten der Lanzette in einer gespannten Position erzeugt und mit einem Auslösemittel die Haltekraft soweit reduziert wird, dass die Lanzette unter Einwirkung der von dem Antriebsmittel erzeugten Antriebskraft in Richtung auf die Hautoberfläche beschleunigt wird.

Lanzettenvorrichtungen werden beispielsweise von Diabetikern benötigt, die ihren Blutzuckerspiegel häufig kontrollieren müssen, um ihn durch Insulininjektionen innerhalb bestimmter Sollgrenzen halten zu können. Durch umfangreiche wissenschaftliche Untersuchungen wurde bewiesen, dass mittels einer Intensivtherapie mit mindestens vier Analysen pro Tag eine dramatische Reduzierung schwerster Spätschäden des Diabetes Mellitus (beispielsweise eine Retinopathie mit resultierender Erblindung des Patienten) erreicht werden kann.

Für Benutzer von Lanzettenvorrichtungen ist einerseits ein möglichst schmerzarmer Einstich und andererseits eine möglichst einfache Bedienung und Handhabbarkeit des verwendeten Lanzettengeräts von großer Bedeutung.

Voraussetzung für einen schmerzarmen Einstich ist eine möglichst schnelle Einstichbewegung mit einer kurzen Verweildauer der Lanzette in der Haut. Im Stand der Technik hat sich für eine entsprechend starke Beschleunigung der Lanzetten die Verwendung von Antriebsfedern bewährt. Ein Nachteil derartiger Lanzettenvorrichtungen liegt darin, dass ein manuelles Spannen der Antriebsfeder nach einem erfolgten Einstich für viele Benutzer mühsam ist. Dies gilt insbesondere für Personen deren manuelle Geschicklichkeit durch Alter oder Krankheit eingeschränkt ist.

Eine Lanzettenvorrichtung, bei der die Antriebsfeder durch einen Elektromotor automatisch gespannt wird, bietet in dieser Hinsicht zwar einen erhöhten Benutzerkomfort, hat jedoch den Nachteil, dass sie wegen des Elektromotors größer und schwerer ist. Eine Lanzettenvorrichtung mit einem integrierten Elektromotor stellt deshalb für Benutzer, die diese für häufige Messungen ständig mit sich führen müssen, eine Belastung dar. Hinzu kommt, dass die Herstellungskosten durch einen Elektromotor wesentlich erhöht werden.

Im Stand der Technik sind ferner Lanzettenvorrichtungen bekannt, bei denen die Antriebskraft auf elektromagnetische Weise mittels einer Spule erzeugt wird. Derartige Lanzettenvorrichtungen sind beispielsweise in der WO 02/100460 A2 und in der US 6364889 B1 offenbart. Um mit derartigen elektromagnetischen Lanzettenantrieben eine für einen schmerzarmen Einstich ausreichend schnelle Einstichbewegung zu bewirken, müssen starke Magnetfelder erzeugt werden. Dies erfordert, dass durch die verwendeten Antriebsspulen relativ starke elektrische Ströme fließen, die sich in einem kleinen, kompakten Handgerät nicht oder nur mit großem Aufwand erzeugen lassen. Elektromagnetische Lanzettenantriebe konnten sich deshalb bisher nicht gegen mechanische Antriebe mit Antriebsfedern durchsetzen.

Eine Lanzettenvorrichtung mit einem elektromagnetischen Antrieb, die diese Nachteile beseitigt, ist aus der WO 2007/006399 A1 bekannt. Die Druckschrift bezieht sich u.a. auf das grundsätzliche Prinzip eines Stechantriebs mit einem Permanentmagneten. Der Antrieb beinhaltet einen Permanentmagneten sowie elektromagnetische Spulen, die eine Kompensation des Permanentmagnetfeldes ermöglichen und somit eine Steuerung der Antriebseinheit für eine Stechhilfe erlauben. Dabei wird die Lanzette durch magnetische Kräfte in einer ersten Position gehalten und durch Kompensation des Magnetfeldes bei einer entsprechenden Bestromung einer Spule kann die Lanzette nicht länger in der Position zurück gehalten werden, so dass die Lanzette von einer Feder angetrieben einen Stechvorgang durchführt. Die aus dieser Druckschrift bekannte Lanzettenvorrichtung, die auch als ballistische Stechhilfe bezeichnet werden kann, weist jedoch wie andere bekannte Ausführungsformen ballistischer Stechhilfen einen Anschlag zum Begrenzen der Stichtiefe auf. Das hat mehrere Konsequenzen und Nachteile:
- Beim Auslösen eines Einstichs entstehen Geräusche, die bis zu einem lauten Klacken reichen können. Diese Geräusche werden von den Benutzern oft als störend empfunden, oder lassen die Benutzer erschrecken, was subjektiv das durch den Einstich verursachte Schmerzempfinden erhöhen kann.
- Es werden in dem Lanzettensystem Schwingungen angeregt, die sich auf die im Gewebe steckende Lanzette übertragen und damit Schmerz verursachen oder erhöhen können.
- Da der Anschlag nicht perfekt inelastisch ist, wird kinetische Energie von dem Anschlag zurück auf die Lanzette übertragen, wodurch das die Lanzette umfassende ballistische System zum Nachschwingen in der Achse der Einstichbewegung neigt. Das kann sogar mehrfachen Lanzettenaustritt zur Folge haben, was ebenfalls Schmerz verursacht.

Um diese unerwünschten Begleiterscheinungen zu unterdrücken, sind nach dem Stand der Technik verschiedene aufwendige Zusatzeinrichtungen erforderlich, welche die Lanzettenvorrichtung oder das gesamte System größer und teurer in der Herstellung machen.

Wenn derartige Stech- bzw. Lanzettenvorrichtungen in ein Blutanalysegerät integriert werden sollen, ist es sinnvoll, die Aktionen "Spannen" und "Auslösen" der Lanzette in die Gesamtergonomie einzubinden, was besonders gut gelingt, wenn sie vom Gerät automatisch betrieben werden, bzw. vom Anwender mit den für ein Analysegerät ohnehin nötigen Bedienelementen gestartet werden können. Zudem lassen sich dann elektrische Bedienelemente (Schalter, Taster, etc.) leichter an ergonomisch sinnvolle Stellen des Geräteäußeren platzieren, als direkt zu greifende mechanische Bedienelemente. Damit steigt der Aufwand für so eine Stecheinrichtung stark an.

Weiterhin sind bahngesteuerte Stechhilfen bekannt (z.B. unter dem Markennamen Softclix), die sich zwar relativ einfach in automatische Meßsysteme integrieren und mit Zusatzfunktionen ausstatten lassen, die aber stets intern Massen in Bewegung setzen, deren kinetische Energie am Ende dissipiert werden muss, was aus funktionalen und aus Platzgründen mit Anschlägen geschieht. Auch diese Systeme verursachen erhebliche Geräusche.

Ferner sind elektrische Antriebe für Stecheinrichtungen bekannt, z.B. aus der oben genannten WO 2002/100460 A2. Diese sind zwar relativ leise, weil sie ohne Endanschläge auskommen, haben aber den Nachteil, dass die volle kinetische Energie für den Stich, incl. der Bewegung der Gerätemassen, die für die Bewegung der Lanzette nötig sind, direkt aus der elektrischen Versorgungsenergie bereitgestellt werden muss. Da für die Erzeugung der benötigten Kräfte große Induktivitäten benötigt werden, diese aber für eine rasche Stromänderung hohe Spannungen benötigen, muss zusätzlich zur Energieversorgung (z.B. Batterie oder Akku) noch eine Leistungsversorgung (z.B. ein Kondensator) im Gerät untergebracht werden, die für hohe Spannungen ausgelegt ist und die nötige Energiemenge für einen Stechvorgang zur Verfügung stellt. So ein Speicher kann aber praktisch nur ca. zur Hälfte genutzt werden. Diese Leistungsversorgung wird dadurch in Bezug auf das Bauvolumen so groß wie die Energieversorgung selbst. Außerdem wird damit der Zwang geschaffen, die bewegten Massen und die Widerstände und Reibungen auf dem Stichweg unbedingt zu minimieren, was Zusatzaufwand für das Entpacken von Stechelementen (Lanzetten) aus der Sterilverpackung erfordert. Beides benötigt Bauvolumen, das in manuell handhabbaren, mobilen Meßsystemen für Diabetiker rar ist.

Aufgabe der Erfindung ist es, einen kostengünstigen Weg aufzuzeigen, wie bei einer Lanzettenvorrichtung der eingangs genannten Art mit einem kompakten Design eine für einen schmerzarmen Einstich ausreichend schnelle Einstichbewegung erzeugt werden kann, wobei störende Geräusche und Erschütterungen möglichst weitgehend vermieden werden und der Benutzer von Vorbereitungshandlungen, wie beispielsweise dem Spannen einer Antriebsfeder, möglichst weitgehend entlastet werden kann.

Diese Aufgabe wird mit einer Lanzettenvorrichtung der eingangs genannten Art erfindungsgemäß dadurch gelöst, dass der Lanzettenantrieb eine Steuereinheit zum Regulieren der Einstichtiefe der Lanzette umfasst, mit der die Reduktion der Haltekraft derart steuerbar ist, dass in Abhängigkeit von der Steuereinheit bei der ausgelösten Einstichbewegung die gewünschte Einstichtiefe der Lanzette resultiert.

Nach einem weiteren, besonders vorteilhaften Merkmal, das bei einer Lanzettenvorrichtung der eingangs genannten Art auch unabhängig von der vorstehend erläuterten Erfindung zweckmäßig verwendet werden kann, wird vorgeschlagen, dass die Lanzette zum freien Ausschwingen in der Einstichrichtung bis zu einem Schwingungsumkehrpunkt ausgebildet ist, so dass die Einstichtiefe der Lanzette, zumindest in einem mittels der Steuereinheit einstellbaren praktisch nutzbaren Einstichtiefenbereich, nicht durch einen mechanischen Einstichtiefenanschlag begrenzt ist.

Die erfindungsgemäße Lanzettenvorrichtung ist ein System, bei dem die zum Ausführen einer Einstichbewegung der Lanzette benötigte Energie bereits in mechanischer Form gespeichert vorliegt, beispielsweise in einer von dem Antriebsmittel umfassten Antriebsfeder, wobei die Antriebsfeder durch die magnetische Haltekraft in einem gespannten Zustand gehalten werden kann, und durch einen elektromagnetischen Aktor gesteuert und moduliert wird, was wesentlich kleinere Leistungen erfordert. Der Lanzettenantrieb umfasst also vorzugsweise einen auf die Bewegung der Lanzette einwirkenden elektromagnetischen Aktor, der von der Steuereinheit zum Regulieren der Einstichtiefe derart ansteuerbar ist, dass die Lanzetteneinstichbewegung mit einem einstellbaren Schub und/oder einer einstellbaren Abbremsung moduliert wird.

Ausgehend von einem Feder-Masse-System mit einer hohen mechanischen Güte, d.h. einer geringen inneren Bedämpfung, kann eine Ausführungsform einer erfindungsgemäßen Lanzettenvorrichtung bevorzugt wie folgt aufgebaut sein bzw. folgende Überlegungen berücksichtigen.

Der Antriebsstößel für eine Lanzette, vorzugsweise eine magazinierte, d.h. in einem Magazin bevorratete und bereitgestellte Lanzette, ist relativ massereich. Er hängt in einer Federanordnung, die es ihm gestattet, von einer zurückgezogenen in eine vorgeschobene Position frei zu schwingen. Die vorderste Position korrespondiert dabei mit der maximalen Austrittsweite der Lanzette. Die Federkonstante der Federanordnung ist so bemessen, dass der Stößel bei Beginn des Austritts der Lanzette eine Geschwindigkeit von ca. 2 m/s aufweist, wenn der Stößel aus der hintersten, gespannten Position gehen gelassen wird.

Weiterhin enthält der Antriebsstößel einen auf oder in dem Stößel fest montierten Permanentmagneten. An die zurückgezogene Position des Stößels anschließend befindet sich ein Elektromagnet mit Eisenkern. Der Magnet im oder an dem Stößel und der Eisenkern sind so dimensioniert, dass der Stößel gegen die Federkraft der Federanordnung in der zurückgezogenen Position gehalten wird, wenn der Stößelmagnet mit dem Eisenkern in Kontakt kommt. Solange der Stößelmagnet aber einen kleinen Abstand von beispielsweise unter 2 mm, bevorzugt weniger als 1,0 mm bis 0,5 mm zum Eisenkern hat, reicht die Anziehungskraft zwischen Eisenkern und Stößelmagnet nicht aus, um den Lanzettenstößel zu halten oder gar nach hinten zu dem Eisenkern zu ziehen.

Entlang eines Teils des Schwingwegs des Stößelmagneten, vorzugsweise entlang des gesamten Schwingwegs des Stößelmagneten, befindet sich eine weitere Spule, die den Bewegungszustand des Stößels detektiert. Evtl. kann die Funktion dieser weiteren Spule aber auch vom Elektromagneten bzw. dessen Spule übernommen werden, so dass diese zusätzliche Spule entfallen kann.

Nach dem Zusammenbau des Gerätes oder evtl. nach einem starken Stoß auf das Gerät befindet sich der Stößel in seiner Ruhelage frei in der Federanordnung hängend, d.h. er liegt nicht an dem Eisenkern des Elektromagneten an. Um das System zu spannen wird der Stößel mit dem Elektromagneten so lange in Schwingung versetzt, bis der Stößelmagnet den Eisenkern erreicht. Vorzugsweise wird dabei der Stößel mit dem Stößelmagneten durch entsprechende Bestromung des Elektromagneten in Resonanz "aufgeschaukelt". Der Eisenkern muss jedoch nicht in Resonanz mit dem Stößelmagneten umgepolt werden, ein Bestromen und Abschalten im richtigen Takt zum Erzeugen einer Beschleunigungskraft in Richtung zu dem Eisenkern reicht auch aus und vereinfacht die zugehörige elektrische Schaltung.

Beim Aufsetzen des Magneten auf den Kern wird eine Spannungsspitze in der Wicklung induziert, die als Signal dient, die Schwingungsanregung abzuschalten. Jetzt ist das Stechsystem mit der nötigen Energie geladen, die Feder ist gespannt und zum Auslösen eines Stechvorgangs bereit.

Um auszulösen wird der Elektromagnet mit einem Strompuls so aktiviert, dass der Eisenkern gegen den Permanentmagneten gepolt wird, so dass die Haltekraft nicht mehr gegen die Federspannung ankommt und der Stößel sich vom Eisenkern löst. Daraufhin schwingt das Feder-Masse-System nach vorne durch, lässt die Lanzette einen ggf. vorhandenen Sterilschutz durchbrechen und sticht die Nadelspitze der Lanzette in die Haut ein. Dabei geht Energie aus dem schwingenden System verloren. Das System ist deshalb vorzugsweise so zu dimensionieren, beispielsweise hinsichtlich der bewegten Massen und der Federspannkraft, dass dieser bei einem Einstichvorgang auftretende Energieverlust, der beispielsweise aufgrund einer variierenden Zähigkeit des Sterilschutzes, einem unterschiedlichen Einstichwiderstand der Haut oder einer unregelmäßigen Schärfe der Nadelspitze auch Schwankungen unterworfen ist, möglichst nur einen kleinen Prozentsatz der Gesamtenergie im System ausmacht. Zu diesem Zweck ist es vorteilhaft, wenn der Stößel massereich ist. Dadurch wird die Austrittsweite aufgrund der genannten Einflussparameter nur sehr wenig verfälscht oder verändert. Dies bedeutet aber auch gleichzeitig, dass die Bewegungsenergie des ausgelösten Stößels beinahe dazu ausreicht, dass der Stößelmagnet bei der Rückführbewegung wieder den Eisenkern erreicht.

Damit der Stößelmagnet auch zuverlässig den Eisenkern wieder erreicht, wird dem schwingenden System vorteilhafterweise etwas Energie zugeführt. Dazu detektiert der Elektromagnet oder ggf. eine zusätzliche Detektorspule die Rückzugsgeschwindigkeit des Stößels und ein Regelprozessor bestromt den Elektromagneten am Ende der Rückschwingung, z.B. in der letzen Hälfte der Rückschwingung, mindestens oder genau so stark, dass der Stößelmagnet sicher, aber ohne nennenswerte Restgeschwindigkeit und damit ohne nennenswerte Geräuschentwicklung auf dem Eisenkern aufsetzt und von diesem wieder gehalten wird.

Die Austrittsweite der Lanzette beim Durchführen einer Einstichbewegung kann dabei entweder mechanisch eingestellt werden, z.B. durch eine einstellbare Hautanlage, relativ zu der die Lanzette austritt, oder durch Steuerung der Bestromung des Elektromagneten beim Auslösen des Einstichs. Beispielsweise wird der Elektromagnet sehr schnell auf Abstoßung des Permanentmagneten geschaltet, dann schwingt das Feder-Masse-System ungehindert los und erreicht die volle Austrittsweite, oder er wird nur langsam auf die nötige auslösende Feldstärke gebracht, so dass der Stößelmagnet auf dem Anfangsweg seiner Laufstrecke nach dem Trennen von dem Eisenkern, beispielsweise auf den ersten 2 mm, noch von einem Rest der Eisenanziehung gebremst wird. Damit verliert das Feder-Masse-System an Energie und schwingt nicht auf die volle Austrittsweite durch. Unterstützt werden kann das durch die Formgebung der Polfläche des Eisenkerns, so dass der Permanentmagnet schon bei Annäherung auf einige mm Abstand einer nennenswerten Anziehung unterliegt, wenn diese auch nicht ausreicht, vollends gegen die Antriebsfeder anzukommen.

Alternativ dazu kann das Feder-Masse-System so ausgelegt werden, dass bei freier Schwingung nur die kleinste einstellbare Austrittsweite erreicht wird. Dann kann die Stärke der Abstoßung durch den Elektromagneten dem Lanzettenstößel den zur gewünschten Austrittsweite benötigten Zusatzschwung geben. Wird dieser Weg gewählt, kann die Polfläche des Eisenkerns eine einfache Planfläche sein.

In beiden Ausführungsformen wird die Austrittsweite per Modulation der Auslösung durch den elektrischen Spannantrieb, d.h. durch eine Steuereinheit bestimmt. Der eigentliche Antrieb bzw. Grundantrieb des Stiches erfolgt durch die Federkraft der Federanordnung, wobei die Stichbewegung durch die Steuereinheit moduliert ist.

In einer weiteren Ausführungsform kann vorgesehen sein, dass zusätzlich ein zweiter Elektromagnet oder ein Permanentmagnet im Bereich des vorderen Totpunktes der Einstichbewegung des Stößelmagneten angeordnet ist, mit dem der Rückzug des Stößels nach dem Erreichen des Umkehrpunktes mehr oder weniger stark gebremst oder in Richtung der Rückzugsbewegung beschleunigt werden kann. Dieser zusätzliche Elektro- oder Permanentmagnet entspricht in seiner Funktion in etwa der eines aus dem Stand der Technik bekannten, die Einstichbewegung begrenzenden Anschlags, dämpft und/oder beschleunigt die Bewegung des Stößels aber ohne Geräusche oder mechanischen Anschlag. Damit sind auch spezielle Bewegungskurven realisierbar.

Bei dieser Ausführungsform ist jedoch die Endposition des Stößels nach dem Stechvorgang eventuell nicht der Eisenkern des hinteren Elektromagneten, sondern die freie Mittelstellung des Feder-Masse-Systems. Diese sollte dann natürlich hinreichend weit vor dem Beginn des Austritts der Lanzette aus dem Gerät angeordnet sein, damit die Lanzette nicht aus dem Gerät herausragt. Außerdem muss in diesem Fall vor Benutzung des Gerätes das Feder-Masse-System erneut in die Ausgangstellung aufgeschwungen werden, woraus ein höherer Energieverbrauch resultiert und überdies der zusätzliche bremsende Elektromagnet beinahe die volle Energie des Stechantriebes aufbringen muss, um diesen zu stoppen. Diese Ausführungsform erzielt daher nicht die vollen erfindungsgemäßen Vorteile.

Mit einer erfindungsgemäßen Vorrichtung ist eine Lanzette entlang eines Einstichweges zum Erzeugen einer Einstichwunde in einer Hautoberfläche, insbesondere zum Gewinnen von Körperflüssigkeit für Diagnosezwecke, verschiebbar. Sie umfasst einen Lanzettenantrieb mit einem Antriebsmittel zum Erzeugen einer Antriebskraft für eine Einstichbewegung der Lanzette entlang des Einstichweges in Richtung auf die Hautoberfläche. Dabei kann der Lanzettenantrieb einen Magneten umfassen, mit dem eine der Antriebskraft entgegen gerichtete magnetische Haltekraft erzeugbar ist, und der Lanzettenantrieb ferner ein Auslösemittel umfassen, mit dem die Haltekraft soweit reduzierbar ist, dass die Lanzette unter Einwirkung der dabei von dem Antriebsmittel erzeugten Antriebskraft in Richtung auf die Hautoberfläche beschleunigt wird.

Als Antriebsmittel kann eine Antriebsfeder verwendet werden, die durch die magnetische Haltekraft in einem gespannten Zustand gehalten werden kann. Bei einer erfindungsgemäßen Lanzettenvorrichtung kann die Lanzette nach dem Eindringen in die Hautoberfläche mittels der magnetischen Haltekraft in ihre Ausgangsposition zurückgezogen werden. Die Haltekraft kann beispielsweise mit einem Elektromagneten erzeugt werden oder von einem Permanentmagneten ausgehen. Wird ein Permanentmagnet verwendet, so ist es vorteilhaft, wenn die Auslösemittel eine Spule umfassen, mit der ein Magnetfeld erzeugbar ist, das die Haltekraft des Permanentmagneten zumindest teilweise, vorzugsweise vollständig, kompensiert. Durch geeignete Dimensionierung des Permanentmagneten und der Spule lässt sich dabei erreichen, dass die von dem Magneten erzeugte Haltekraft ausreichend groß ist, um nach einem erfolgten Einstich ein erneutes Spannen der Antriebsfeder zu bewirken.

Als Antriebsmittel kann für eine erfindungsgemäße Lanzettenvorrichtung ferner eine Spule verwendet werden, um die Antriebskraft auf magnetischem Wege zu erzeugen. Die Spule kann zugleich auch als Auslösemittel verwendet werden, mit dem die magnetische Haltekraft überkompensiert wird. Bevorzugt wird die magnetische Haltekraft von einem Permanentmagneten erzeugt, dem als zweiter Teil des Antriebsmittels ein weiterer Permanentmagnet als Antriebsmagnet mit umgekehrter Polarisierung zugeordnet ist, so dass sich die Magnetfelder der Permanentmagnete destruktiv überlagern. Auf diese Weise wird die von dem Antriebsmagneten erzeugte Antriebskraft von dem die Haltekraft erzeugenden Permanentmagneten kompensiert, so dass sich ohne Spulenstrom keine resultierende Antriebskraft und folglich auch keine Lanzettenbewegung ergibt. Fließt durch die Antriebsspule ein Strom, überlagert sich das Magnetfeld des Antriebsmagneten konstruktiv mit dem Magnetfeld der Spule, so dass eine resultierende Antriebskraft zur Beschleunigung einer Lanzette entsteht.

Selbst wenn sich die beiden Permanentmagnete exakt kompensieren, kann verblüffenderweise durch die Verwendung einer Antriebsspule in Kombination mit entgegen gesetzt polarisierten Permanentmagneten eine größere Antriebskraft erzeugt werden als mit einer Antriebsspule für sich alleine. Durch die Überlagerung des Spulenfeldes mit den Feldern der entgegen gesetzt polarisierten Permanentmagneten ergibt sich nämlich lokal in einem ersten Bereich eine erhöhte magnetische Feldstärke und in einem zweiten Bereich eine lokal reduzierte Feldstärke. Die lokal erhöhte Magnetfeldstärke kann dazu benutzt werden, ein Antriebselement, beispielsweise einen weichmagnetischen Spulenkern, zu magnetisieren. Die von dem Magnetfeld auf das Antriebselement ausgeübte Kraft ist dann wegen der lokal erhöhten Feldstärke insgesamt größer als bei Verwendung einer Spule ohne Permanentmagnete.

Die Erfindung wird nachfolgend anhand in den Figuren dargestellter Ausführungsbeispiele näher erläutert. Die darin dargestellten Besonderheiten können einzeln oder in Kombination verwendet werden, um bevorzugte Ausgestaltungen der Erfindung zu schaffen. Es zeigen:
- Fig. 1: ein erstes Ausführungsbeispiel einer erfindungsgemäßen Lanzettenvorrichtung mit entspannter Antriebsfeder;
- Fig. 2: das in Fig. 1 gezeigte Ausführungsbeispiel mit gespannter Antriebsfeder;
- Fig. 3: das in Fig. 1 gezeigte Ausführungsbeispiel mit ausgeschwungener Lanzette beim Einstechen in einen Körper;
- Fig. 4: den Spannungsverlauf in der Spule des in Fig. 1 gezeigten Ausführungsbeispiels; und
- Fig. 5: ein zweites Ausführungsbeispiel einer erfindungsgemäßen Lanzettenvorrichtung mit entspannter Antriebsfeder.

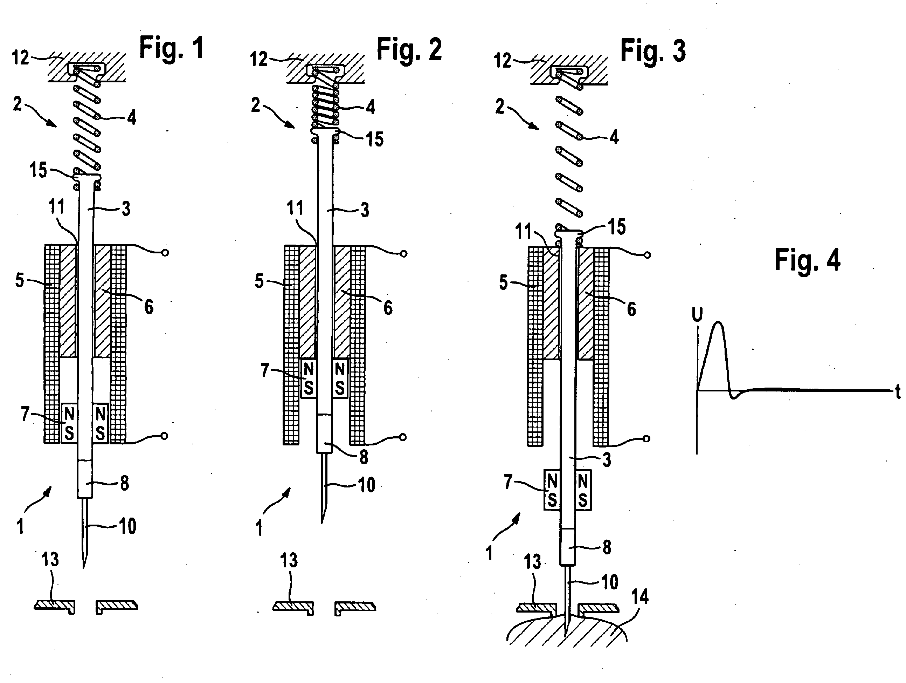

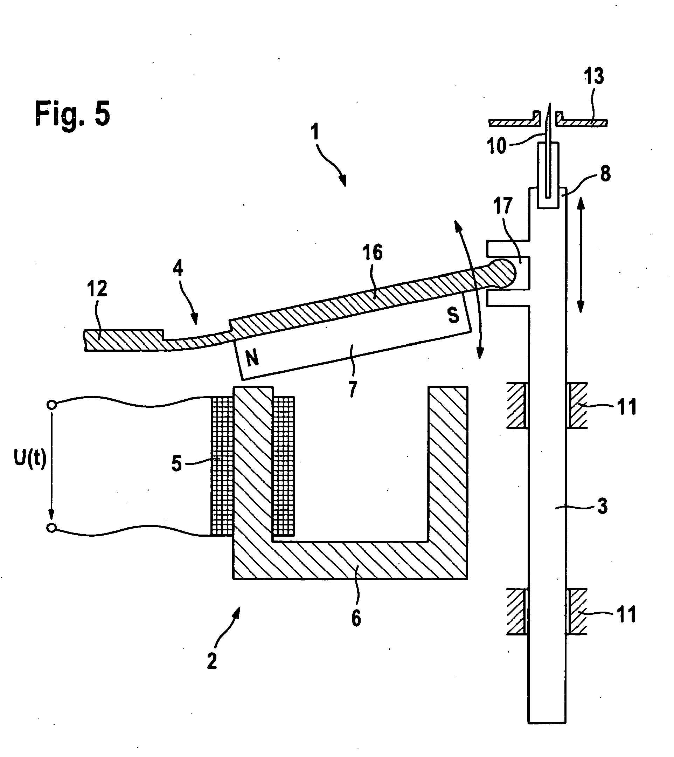

Die in den Fig. 1 bis 3 und in Fig. 5 dargestellten Lanzettenvorrichtungen 1 können beispielsweise in ein Analysehandgerät integriert werden, das eine Messeinrichtung zum Untersuchen von Körperflüssigkeit, die aus einer erzeugten Einstichwunde gewonnen wird, aufweist. Ebenso gut können die dargestellten Lanzettenvorrichtungen in ein separates Gerät als Stechhilfe eingebaut werden.

Zentraler Bestandteil der in den Fig. 1 bis 3 dargestellten Lanzettenvorrichtung 1 ist ein Lanzettenantrieb 2, der eine Schubstange 3, die auch als Lanzettenführung bezeichnet werden kann, ein Antriebsmittel in Form einer Antriebsfeder 4 oder einem Paar Federn für Vor- und Rückhub, eine Spule 5 mit einem Eisenjoch 6 und einen axial in der Spule 5 angeordneten Permanentmagneten 7 umfasst. Die Schubstange 3 ist bevorzugt aus einem magnetisch neutralen Werkstoff, weiter bevorzugt aus einem nicht elektrisch leitenden Werkstoff und insbesondere bevorzugt aus Kunststoff, um Wirbelstromverlustes bei der Bewegung im Magnetfeld zu vermeiden, und trägt diesen Permanentmagneten 7 bzw. Stößelmagneten 7 und einen Lanzettenhalter 8 mit einer austauschbaren Lanzette 10 und wird zum Erzeugen einer Einstichwunde mit der als Schraubenfeder ausgebildeten Antriebsfeder 4 entlang eines durch die Führung 11 vorgegebenen Einstichwegs beschleunigt. In alternativen Ausführungsformen kann die Schubstange 3 eine Ankerplatte aus Eisen oder einem anderen ferromagnetischen Material tragen, mit der in der in Fig. 2 gezeigten gespannten Position ein magnetischer Kreis geschlossen wird, in Fig. 2 mit dem Eisenjoch 6 und ggf. einem die Spule 6 umgebenden Polschuh. Wenn die Ankerplatte als Permanentmagnet (Stößelmagnet 7) ausgebildet ist, kann sie nicht nur von dem Eisenjoch 6 angezogen, sondern auch mit einem von der Spule 5 erzeugten Magnetfeld abgestoßen und zusätzlich beschleunigt werden.

Die Lanzette 10 ist abnehmbar an dem Lanzettenhalter 8 auf der Schubstange 3 befestigt. An dieser ist fest angebracht der Stößelmagnet 7. Allgemein kann vorgesehen sein, dass der Lanzettenantrieb 2 eine Ankerplatte umfasst, die auf einer die Lanzette 10 tragenden Schubstange 3 angeordnet und aus Eisen, einem anderen ferromagnetischen Material oder als Permanentmagnet ausgebildet ist. Korrespondierend zum Magneten 7 (hier koaxial um die Führung 11) sind ein Eisenjoch 6 und eine Spule 5 gestellfest montiert. Die Schubstange 3 ist mit einer Feder 4 oder einem Paar Federn für Vor- und Rückhub ausgestattet, die mit ihrem festen Ende am Gestell 12 befestigt ist. Ebenfalls am Gestell 12 ist die Hautanlage 13 angeordnet. Die Spule 5 dient als Rückhaltespule des Rückhaltemittels und hat einen magnetisierbaren oder als Permanentmagnet ausgebildeten Spulenkern 6. Die Schubstange 3 ragt in den von dem Eisenjoch 6 gebildeten Spulenkern, der eine Führung 11 für die Schubstange 3 bildet, hinein.

Der Lanzettenantrieb 2 kann neben der Antriebsfeder 4 ferner eine Rückholfeder zum Erzeugen einer Rückführbewegung der Lanzette 10 umfassen. Mit der Rückführbewegung wird die Antriebsfeder 4 erneut gespannt. Antriebsfeder 4 und Rückholfeder können jeweils als Schraubenfedern ausgebildet sein, welche die Schubstange 3 umgeben. Die Antriebsfeder 4 und ggf. die Rückholfeder stützen sich jeweils mit einem Ende an einem Stützabschnitt der Schubstange 3, der bei dem dargestellten Ausführungsbeispiel als Verdickung 15 ausgeführt ist, und mit dem jeweils anderen Ende an dem Gestell ab. Die Antriebsfeder 4 und die Rückholfeder können so angeordnet sein, dass ein Entspannen der Antriebsfeder 4 ein Spannen der Rückholfeder und ein Entspannen der Rückholfeder ein Spannen der Antriebsfeder 4 bewirkt. Der Begriff "spannen" ist in diesem Zusammenhang so zu verstehen, dass in der jeweils betroffenen Feder Energie gespeichert wird. Dies kann beispielsweise bei einer Kompressionsfeder durch Zusammendrücken, bei einer Dehnungsfeder durch Auseinanderziehen oder bei einer Blattfeder durch Biegen bewirkt werden.

In anderen Ausführungsformen kann das Antriebsmittel zusätzlich oder anstelle der Antriebsfeder 4 auch eine Antriebsspule mit einem magnetisierbaren oder als Permanentmagnet ausgebildeten Spulenkern umfassen. In Fig. 1 kann beispielsweise auch die Spule 5 mit dem Eisenjoch 6 die Funktion des Antriebsmittels übernehmen oder dazu beitragen. Ferner kann vorgesehen sein, dass die Antriebsspule und die Rückhaltespule dieselbe Spule sind.

In Fig. 1 ist die Lanzettenvorrichtung mit entspannter Antriebsfeder 4 dargestellt. Zum Spannen der Lanzettenvorrichtung 1 wird durch rückgekoppelte Wechselbestromung der Spule 5 das Feder-Masse-System aus Stößel 3 und Feder 4 aufgeschaukelt, bis der Stößelmagnet 7 in Kontakt mit dem Eisenjoch 6 kommt (Fig. 2). In diesem Zustand kann die Spule 5 stromlos bleiben, der Stößelmagnet 7 hält sich gegen die gespannte Feder 4 am Eisenjoch 6 fest.

In Fig. 2 ist die Schubstange 3 in ihrer Position mit gespannter Antriebsfeder 4 dargestellt. Die Antriebsfeder 4 ist eine Schraubenfeder, die auf eine die Lanzette 10 tragende Schubstange 3 einwirkt Die Schubstange 3 bzw. die Antriebsfeder 4 wird in dieser Position durch die magnetische Haltekraft des in den Spulenkern der Spule 5 integrierten Eisenjochs 6 oder eines Permanentmagneten gehalten. Der Spulenkern ist ortsfest hinsichtlich der Spule 5 angeordnet. Die Lanzettenvorrichtung 1, mit der die Lanzette 10 entlang eines Einstichweges zum Erzeugen einer Einstichwunde in einer Hautoberfläche, insbesondere zum Gewinnen von Körperflüssigkeit für Diagnosezwecke, verschiebbar ist, umfasst also einen Lanzettenantrieb 2, der ein Antriebsmittel zum Erzeugen einer Antriebskraft für eine Einstichbewegung der Lanzette entlang des Einstichweges in Richtung auf die Hautoberfläche umfasst, ein Rückhaltemittel zum Erzeugen einer magnetischen Haltekraft zum Halten der Lanzette 10 in einer gespannten Position, und ein Auslösemittel, mit dem die Haltekraft soweit reduzierbar ist, dass die Lanzette unter Einwirkung der von dem Antriebsmittel erzeugten Antriebskraft in Richtung auf die Hautoberfläche beschleunigt wird, und weist eine Steuereinheit zum Regulieren der Einstichtiefe der Lanzette 10 auf, mit der die Reduktion der Haltekraft derart steuerbar ist, dass in Abhängigkeit von der Steuereinheit bei der ausgelösten Einstichbewegung die gewünschte Einstichtiefe der Lanzette 10 resultiert.

Die Spule 5 dient als Auslösemittel zum Auslösen einer Einstichbewegung. Indem man durch die Spule 5 einen elektrischen Strom fließen lässt, wird ein Magnetfeld erzeugt, das die Haltekraft des Stößelmagneten 7 kompensiert, so dass sich die Antriebsfeder 4 entspannt und die Schubstange 3 mit der Lanzette 10 unter Einwirkung der dabei von der Antriebsfeder 4 erzeugten Antriebskraft in Richtung auf die Hautoberfläche beschleunigt wird.

Der Einstechvorgang ist in Fig. 3 dargestellt. Für den Stich wird die Spule 5 mit einem Strompuls beaufschlagt, so dass die Haltekraft des Permanentmagneten 7 am Eisenjoch 6 überwunden wird. Die Einstichtiefe in die Haut 14 kann von einer Steuereinheit über die Stärke und Dauer dieses Pulses gesteuert werden. Ggf. kann auch die Geschwindigkeit des Rückzuges verlangsamt werden, indem zu gegebener Zeit gegenbestromt wird.

Die Steuerung braucht dazu keinen Wegsensor oder sonst eine Lageerkennung, sondern lediglich eine Information über die Geschwindigkeit und die Bewegungsrichtung, die z.B. über die Gegeninduktion in der Spule 5 erfasst werden kann.

Durch eine geregelte "Bestromung" ist ein Spannen der Feder sowie eine Variation der Einstichtiefe möglich. Die Auslösung kann dazu benutzt werden, die Stichweite zu modulieren (z.B. durch einen elektromagnetischen Schub für größere Austrittsweiten und/oder eine Bremsung für geringere Austrittsweiten. Die Spule 5 dient sozusagen als auf die Bewegung der Lanzette 10 einwirkender elektromagnetischer Aktor des Lanzettenantriebs 2, wobei der Aktor von der Steuereinheit zum Regulieren der Einstichtiefe derart ansteuerbar ist, dass die Lanzetteneinstichbewegung mit einem einstellbaren Schub und/oder einer einstellbaren Abbremsung moduliert wird.

Auch in einem Störfall besteht die Möglichkeit, die Schubstange 3 aus einer in Fig. 1 dargestellten Zwischenposition wieder in die gespannte Ausgangsposition gemäß Fig. 2 zu bewegen, indem mittels der Spule 5 durch periodische Stromstöße eine mechanische Schwingung des von der Antriebsfeder 4, der Schubstange 3 und ggf. einer Rückholfeder gebildeten mechanischen Systems angeregt wird. Bei fortwährender Anregung mit der Resonanzfrequenz dieses Systems nehmen die Amplituden dieser Schwingung zu, bis die Schubstange 3 in die Start- bzw. Ausgangsposition gemäß Fig. 2 zurückkehrt und dort von der magnetischen Haltekraft in der gespannten Position, nämlich durch die Haltekraft des Stößelmagneten 7 an dem Eisenjoch 6 gehalten werden kann.

Durch eine Messung der Induktivität der Spule 5 kann festgestellt werden, ob der Stößelmagnet 7 an dem Eisenjoch anliegt. Auf diese Weise kann also ermittelt werden, ob sich die Schubstange 3 in der in Figur 2 dargestellten gespannten Position befindet oder nicht. Bevorzugt wird kurz nach einem Einstich, beispielsweise 1 bis 2 Sekunden nach einem Einstich, eine Messung der Induktivität der Spule 5 durchgeführt. Falls dabei festgestellt wird, dass sich die Schubstange 3 nicht in der gespannten Position befindet, wird durch periodische Stromstöße eine mechanische Schwingung des von der Antriebsfeder 4 und der Schubstange 3 gebildeten mechanischen Systems angeregt, so dass die Schubstange 3 in ihre gespannte Position zurückkehrt.

Die Spule 5 wird auf diese Weise als Positionssensor für die Position der Schubstange 3 verwendet. Die dargestellte Lanzettenvorrichtung 1 kann alternativ oder zusätzlich auch mit anderen Positionssensoren ausgerüstet werden, so dass der optimale Zeitpunkt zum Abschalten oder Umpolen des Stroms durch die Spule 5 in Abhängigkeit von der momentanen Position der Schubstange 3 ermittelt werden kann. Der Einsatz von Sensoren ermöglicht deshalb anstelle einer einfachen Steuerung des Spulenstroms, bei dem ein vorgegebenes Profil für den Strompuls vorgegeben wird, eine Regelung des Spulenstroms in Abhängigkeit von der Position der Schubstange 3.

Allgemein kann es vorteilhaft sein, wenn mit dem elektromagnetischen Aktor ein Magnetfeld erzeugbar ist, das die Haltekraft teilweise kompensiert, vollständig kompensiert oder überkompensiert, um die Einstichtiefe der Lanzette 10 zu steuern. In vorteilhaften Ausführungsformen umfasst der elektromagnetische Aktor eine Aktorspule, ggf. mit einem magnetisierbaren oder als Permanentmagnet ausgebildeten Spulenkern. In bevorzugten Ausführungsformen sind mindestens zwei Spulen der Antriebsspule, der Rückhaltespule und der Aktorspule dieselbe Spule, um Bauteile einzusparen.

Weiterhin ist kein mechanischer Anschlag für die Begrenzung der Austrittsweite der Lanzette 10 vorgesehen, wie er von ballistischen Stechhilfen bislang bekannt ist. Stattdessen ist das schwingfähige System aus Schubstange 3 und Lanzette 10 so ausgelegt, dass die Lanzette 10 bis zu ihrem Schwingungsumkehrpunkt durchschwingt. Die Schubstange 3 mit der Lanzette 10 ist zum freien Ausschwingen in der Einstichrichtung bis zu einem Schwingungsumkehrpunkt ausgebildet, so dass die Einstichtiefe der Lanzette 10, zumindest in einem mittels der Steuereinheit einstellbaren praktisch nutzbaren Einstichtiefenbereich, nicht durch einen mechanischen Einstichtiefenanschlag begrenzt ist. In Fig. 3 ist die Lanzettenvorrichtung 1 mit der Schubstange 3 in der Einstichposition dargestellt. In der Einstichposition liegt kein beispielsweise von dem Stößelmagneten 7 gebildeter Anschlag an einem Begrenzungselement der Führung 11 an, so dass der Einstichweg nicht dadurch begrenzt wird. Natürlich erreicht das dargestellte System aus Schubstange 3 und Stößelmagnet 7 bei einer weiteren, über den normalen Betrieb hinausgehenden Bewegung in der Einstichrichtung irgendwann einen mechanischen Anschlagpunkt, der die weitere Bewegung begrenzt. So kann beispielsweise zum Verhindern eines Herausziehens der Schubstange 3 durch einen Benutzer die Schubstange 3 eine Verdickung 15 aufweisen, die an dem Eisenjoch 6 anstößt. Beim dem bestimmungsgemäßen Gebrauch der Lanzettenvorrichtung 1 wird jedoch das Ausschwingen nicht durch diesen Anschlag begrenzt.

Ferner kann vorgesehen sein, dass die einmal durch Aufschwingen im System enthaltene Energie in der Regel erhalten bleibt. Einmal gehengelassen schwingt der Stößel 3 hin und her. Auf dem Rückweg ist nur wenig Energie nötig, um ihn wieder einzufangen. Dies bedeutet, dass die überschüssige Energie nicht wie bislang üblich dissipiert wird (z.B. mit Dämpfern in Wärme umgewandelt), sondern als Federspannung gespeichert wird bis zum nächsten Stich. Nur im Versagensfall des Haltemagneten muss sie erneut in das System geschaukelt werden, wobei nach einem erfolgten Einstich mittels der Steuereinheit durch periodische Stromstöße eine mechanische Schwingung der Lanzette 10 angeregt wird, durch welche eine die Lanzette 10 tragende Schubstange 3 in ihre Ausgangsposition zurückkehrt, in der sie von der magnetischen Haltekraft in der gespannten Position gehalten wird. Der Lanzettenantrieb 2 umfasst also vorteilhafterweise einen Aktor, der mittels der Steuereinheit derart steuerbar ist, dass die Lanzette 10 nach dem Durchführen eines Einstichs in einer der Einstichbewegung entgegengesetzten Rückführbewegung derart beschleunigbar ist, dass die Lanzette 10 in die Ausgangslage zurückkehrt, in der sie von der magnetischen Haltekraft in der gespannten Position gehalten wird.

Selbstverständlich treten bei der dargestellten Lanzettenvorrichtung 1 Reibungskräfte auf, so dass die in der in Fig. 3 dargestellten Einstichposition, ggf. auch in einer zusätzlichen Rückholfeder gespeicherte Energie nicht ganz zum erneuten Spannen der Antriebsfeder 4 ausreicht. Bei der dargestellten Vorrichtung wird deshalb die Rückführbewegung der Schubstange 3 von der Steuereinheit unterstützt. Die von der Spule 5 auf den Stößelmagneten 7 in der Rückführbewegung ausgeübte Beschleunigung reicht aus, um die Schubstange 3 erneut in die in Fig. 2 dargestellte gespannte Position zu bringen und dabei die Antriebsfeder 4 zu spannen.

Um die Rückführbewegung zu unterstützen kann die Richtung des zum Auslösen eines Einstichs durch die Spule 5 fließenden Stroms umgepolt werden, so dass sich das von der Spule 5 erzeugte Magnetfeld zu der Haltekraft des Stößelmagneten 7 addiert. Zum Umpolen des Stroms kann beispielsweise eine Steuereinheit mit einer H-Brücke verwendet werden. Beispielsweise erfolgt das Umpolen in dem Moment, in dem die Lanzette 10 den äußersten Punkt des Einstichwegs erreicht hat.

Eine erfindungsgemäße Lanzettenvorrichtung 1 ist also nach einem vorteilhaften zusätzlichen Merkmal derart ausgebildet, dass der Lanzettenantrieb 2 und die Lanzette 10, insbesondere eine die Lanzette 10 tragende Schubstange 3, die Lanzette 10 und das Antriebsmittel, das in den dargestellten Ausführungsbeispielen die Antriebsfeder 4 ist, ein schwingendes System bilden, das entlang des Einstichweges schwingen kann. Dabei ist vorteilhafterweise vorgesehen, dass die bei dem Durchführen eines Einstichs mit der Lanzette 10 in dem Schwingungssystem enthaltene Schwingungsenergie zu einem überwiegenden Teil durch ein im Anschluss an den Einstich erfolgendes erneutes Spannen des Antriebsmittels in einer Ausgangsstellung des Antriebsmittels als potentielle Energie wiedergewonnen und gespeichert wird, so dass die wiedergewonnene kinetische Energie zum Durchführen eines folgenden Einstichs zur Verfügung steht und nicht neu aufgebracht werden muss. Die zum Durchführen eines Einstichs erforderliche Energie beträgt ca. 1,0 mJ bis 5,0 mJ, typischerweise 2,5 mJ. Die Schwingungsenergie des schwingenden Systems ist vorteilhafterweise mindestens 10x, bevorzugt mindestens 50x und besonders bevorzugt mindestens 100x so groß wie die erforderliche Einstichenergie. Die typische Schwingungsenergie liegt somit zwischen 25 mJ und 250 mJ.

Eine Einstich- und Rückführbewegung der Lanzette 10 dauert insgesamt typischerweise 4 ms bis 6 ms. Für eine möglichst schnelle Einstichbewegung ist eine entsprechend starke Antriebsfeder 4 und ein entsprechend starker Stößelmagnet 7, beispielsweise ein Seltenerdmagnet, bevorzugt. Zur Kompensation der magnetischen Haltekraft werden deshalb bevorzugt Spannungen und/oder Stromstärken verwendet, die weit über die Leistungsfähigkeit handelsüblicher Batterien hinausgehen. Bevorzugt ist die Spule 5 deshalb über Strompuffer und/oder Spannungswandler an eine interne Stromquelle der Lanzettenvorrichtung 1, beispielsweise eine Batterie, angeschlossen, so dass von der Steuereinheit mit handelsüblichen Batterien ein zum Kompensieren der magnetischen Haltekraft geeigneter Strompuls durch die Spule 5 erzeugt werden kann. Als Strompuffer sind beispielsweise Kondensatoren oder Akkumulatoren, insbesondere Lithium-Polymer-Akkumulatoren, geeignet. Geeignete Spannungswandler sind als DC/DC-Konverter verfügbar. Die entsprechende Technologie zum Erzeugen intensiver Strompulse ist beispielsweise zum Erzeugen von Lichtblitzen bei Fotoapparaten gebräuchlich und kann für die beschriebene Lanzettenvorrichtung 1 genutzt werden.

Die Fig. 4 zeigt den Spannungsverlauf in der Spule 5 des in Fig. 1 gezeigten Ausführungsbeispiels.

In Fig. 5 ist ein abgewandeltes Ausführungsbeispiel einer erfindungsgemäßen Lanzettenvorrichtung 1 dargestellt. Es weist eine "relais-artige" Ausführung des Lanzettenantriebs 2 auf, bei der die Spule 5 auf einem u-förmigen Eisenjoch 6 angeordnet ist, wobei die Führung 11 des linear hin und her verschiebbaren bzw. schwingenden Stößels 3 nicht von dem Eisenjoch 6 gebildet, sondern separat davon angeordnet ist. Die Antriebsfeder 4, die auf die die Lanzette 10 tragende Schubstange 3 einwirkt, ist in diesem Fall keine Schraubenfeder, sondern eine Blattfeder. Mit einem Ende ist sie fest am Gestell 12 eingespannt und an dem anderen Ende ist sie mit einem Mitnehmer 16 verbunden, der in eine Aufnahme 17 des Stößels 3 eingreift. Selbstverständlich können zusätzlich zu der Blattfeder auch Schraubenfedern, die an dem Mitnehmer 16, der Aufnahme 17 oder dem Stößel 3 angreifen, vorgesehen sein.

Der Mitnehmer 16 trägt einen Permanentmagneten 7, der von dem Eisenjoch 6 angezogen oder abgestoßen werden kann, und zwar in Abhängigkeit von der Ansteuerung der Spule 5 mit der Spannung U, die in Abhängigkeit von der Zeit t variiert wird.

Die weitere Ausgestaltung des in Fig. 5 dargestellten zweiten Ausführungsbeispiels kann entsprechend zu der in den Figuren 1 bis 3 dargestellten ersten Ausführungsform getroffen sein. In Fig. 5 ist die Lanzettenvorrichtung mit entspannter Antriebsfeder 4 dargestellt. Zum Spannen der Lanzettenvorrichtung 1 wird mittels der Spule 5 und des Eisenjochs 6 ein Magnetfeld erzeugt, durch das der Permanentmagnet 7 angezogen wird, bis er in Kontakt mit dem Eisenjoch 6 kommt. In diesem Zustand kann die Spule 5 stromlos bleiben, der Permanentmagnet 7 hält sich gegen die gespannte Feder 4 am Eisenjoch 6 fest.

Die Spule 5 dient auch als Auslösemittel zum Auslösen einer Einstichbewegung. Indem man durch die Spule 5 einen elektrischen Strom fließen lässt, wird ein Magnetfeld erzeugt, das die Haltekraft des Permanentmagneten 7 kompensiert, so dass sich die Antriebsfeder 4 entspannt und die Schubstange 3 mit der Lanzette 10 unter Einwirkung der von der Antriebsfeder 4 über den Mitnehmer 6 und die Aufnahme 17 auf die Schubstange 3 erzeugten Antriebskraft in Richtung auf die Hautoberfläche beschleunigt wird. Die Einstichtiefe in die Haut kann auch hierbei von einer Steuereinheit über die Stärke und Dauer des auf die Spule 5 aufgeprägten Pulses gesteuert werden. Ebenso ist es möglich, sofern erforderlich, durch eine geeignete Bestromung das schwingungsfähige System zum Spannen aufzuschaukeln. Im übrigen wird auf die Ausführungen zu den Fig. 1 bis 3 Bezug genommen.

### Bezugszeichenliste

- 1: Lanzettenvorrichtung
- 2: Lanzettenantrieb
- 3: Schubstange
- 4: Antriebsfeder
- 5: Spule
- 6: Eisenjoch
- 7: Permanentmagnet
- 8: Lanzettenhalter
- 10: Lanzette
- 11: Führung
- 12: Gestell
- 13: Hautanlage
- 14: Haut
- 15: Verdickung
- 16: Mitnehmer
- 17: Aufnahme
- U: Spannung
- t: Zeit

## Patentansprüche

1. Lanzettenvorrichtung (1), mit der eine Lanzette (10) entlang eines Einstichweges zum Erzeugen einer Einstichwunde in einer Hautoberfläche, insbesondere zum Gewinnen von Körperflüssigkeit für Diagnosezwecke, verschiebbar ist, umfassend
einen Lanzettenantrieb (2), der ein Antriebsmittel zum Erzeugen einer Antriebskraft für eine Einstichbewegung der Lanzette (10) entlang des Einstichweges in Richtung auf die Hautoberfläche umfasst,
ein Rückhaltemittel zum Erzeugen einer magnetischen Haltekraft zum Halten der Lanzette (10) in einer gespannten Position, und
ein Auslösemittel, mit dem die Haltekraft soweit reduzierbar ist, dass die Lanzette (10) unter Einwirkung der von dem Antriebsmittel erzeugten Antriebskraft in Richtung auf die Hautoberfläche beschleunigt wird,
**dadurch gekennzeichnet, dass**
der Lanzettenantrieb eine Steuereinheit zum Regulieren der Einstichtiefe der Lanzette (10) umfasst, mit der die Reduktion der Haltekraft derart steuerbar ist, dass in Abhängigkeit von der Steuereinheit bei der ausgelösten Einstichbewegung die gewünschte Einstichtiefe der Lanzette (10) resultiert.

2. Lanzettenvorrichtung (1) nach Anspruch 1, **dadurch gekennzeichnet, dass** die Lanzette (10) zum freien Ausschwingen in der Einstichrichtung bis zu einem Schwingungsumkehrpunkt ausgebildet ist, so dass die Einstichtiefe der Lanzette (10), zumindest in einem mittels der Steuereinheit einstellbaren praktisch nutzbaren Einstichtiefenbereich, nicht durch einen mechanischen Einstichtiefenanschlag begrenzt ist.

3. Lanzettenvorrichtung (1) nach einem der vorhergehenden Ansprüche, **dadurch gekennzeichnet, dass** das Antriebsmittel eine Antriebsfeder (4) umfasst, wobei die Antriebsfeder (4) durch die magnetische Haltekraft in einem gespannten Zustand gehalten werden kann.

4. Lanzettenvorrichtung (1) nach einem der vorhergehenden Ansprüche, **dadurch gekennzeichnet, dass** der Lanzettenantrieb (2) eine Ankerplatte umfasst, die auf einer die Lanzette (10) tragenden Schubstange (3) angeordnet und aus Eisen, einem anderen ferromagnetischen Material oder als Permanentmagnet ausgebildet ist.

5. Lanzettenvorrichtung (1) nach einem der vorhergehenden Ansprüche, **dadurch gekennzeichnet, dass** der Lanzettenantrieb (2) einen auf die Bewegung der Lanzette (10) einwirkenden elektromagnetischen Aktor umfasst, der von der Steuereinheit zum Regulieren der Einstichtiefe derart ansteuerbar ist, dass die Lanzetteneinstichbewegung mit einem einstellbaren Schub und/oder einer einstellbaren Abbremsung moduliert wird.

6. Lanzettenvorrichtung (1) nach dem vorhergehenden Anspruch, **dadurch gekennzeichnet, dass** mit dem elektromagnetische Aktor ein Magnetfeld erzeugbar ist, das die Haltekraft teilweise kompensiert, vollständig kompensiert oder überkompensiert.

7. Lanzettenvorrichtung (1) nach einem der Ansprüche 5 bis 6, **dadurch gekennzeichnet, dass** der Aktor mittels der Steuereinheit derart steuerbar ist, dass die Lanzette (10) nach dem Durchführen eines Einstichs in einer der Einstichbewegung entgegengesetzten Rückführbewegung derart beschleunigbar ist, dass die Lanzette (10) in die Ausgangslage zurückkehrt, in der sie von der magnetischen Haltekraft in der gespannten Position gehalten wird.

8. Lanzettenvorrichtung (1) nach einem der vorhergehenden Ansprüche, **dadurch gekennzeichnet, dass** der Lanzettenantrieb (2) und die Lanzette (10), insbesondere eine die Lanzette (10) tragende Schubstange (3), die Lanzette (10) und das Antriebsmittel, insbesondere eine Antriebsfeder (4), ein schwingendes System bilden, das entlang des Einstichweges schwingen kann.

9. Lanzettenvorrichtung (1) nach dem vorhergehenden Anspruch, **dadurch gekennzeichnet, dass** sie derart ausgebildet ist, dass die bei dem Durchführen eines Einstichs mit der Lanzette (10) in dem Schwingungssystem enthaltene Schwingungsenergie zu einem überwiegenden Teil durch ein im Anschluss an den Einstich erfolgendes erneutes Spannen des Antriebsmittels in einer Ausgangsstellung des Antriebsmittels als potentielle Energie wiedergewonnen und gespeichert wird, so dass die wiedergewonnene kinetische Energie zum Durchführen eines folgenden Einstichs zur Verfügung steht und nicht neu aufgebracht werden muss.

10. Lanzettenvorrichtung (1) nach einem der Ansprüche 8 bis 9, **dadurch gekennzeichnet, dass** sie derart ausgebildet ist, dass die beim Durchführen eines Einstichs mit der Lanzette (10) in dem Schwingungssystem enthaltene Schwingungsenergie wesentlich größer ist als die Energie, die zum Durchführen eines Einstichs in die Haut erforderlich ist.

11. Analysegerät zum Analysieren eines medizinisch bedeutsamen Bestandteils einer Körperflüssigkeit oder Stechhilfe zum Entnehmen einer Probe einer Körperflüssigkeit durch einen Einstich in einen Körper, **dadurch gekennzeichnet, dass** das Analysegerät bzw. die Stechhilfe eine Lanzettenvorrichtung (1) nach einem der Ansprüche 1 bis 10 umfasst.

## Claims

1. A lancet device (1), by which a lancet (10) can be displaced along a piercing path to generate a piercing wound in a skin surface, in particular to obtain bodily fluid for diagnostic purposes, comprising
a lancet drive (2), which has drive means for generating a drive force for a piercing movement of the lancet (10) along the piercing path in the direction toward the skin surface, comprising
retention means for generating a magnetic retention force for retaining the lancet (10) in a tensioned position, and
triggering means, using which the retention force is reducible enough that the lancet (10) is accelerated in the direction toward the skin surface under the effect of the drive force generated by the drive means,
**characterized in that**
the lancet drive comprises a control unit for regulating the piercing depth of the lancet (10), by which the reduction of the retention force is controllable in such a way that the desired piercing depth of the lancet (10) results as a function of the control unit during the triggered piercing movement.

2. The lancet device (1) according to claim 1, **characterized in that** the lancet (10) is implemented to oscillate freely in the piercing direction up to an oscillation reversal point so that the piercing depth of the lancet (10), at least in a practically usable piercing depth range settable using the control unit, is not delimited by a mechanical piercing depth stop.

3. The lancet device (1) according to any one of the preceding claims, **characterized in that** the drive means comprises a drive spring (4), wherein the drive spring (4) can be retained in a tensioned state by the magnetic retention force.

4. The lancet drive (1) according to any one of the preceding claims, **characterized in that** the lancet drive (2) comprises an armature plate, which is situated on a pushrod (3) carrying the lancet (10) and is implemented from iron, another ferromagnetic material, or as a permanent magnet.

5. The lancet device (1) according to any one of the preceding claims, **characterized in that** the lancet drive (2) comprises an electromagnetic actuator acting on the movement of the lancet (10), which is drivable by the control unit to regulate the piercing depth in such a way that the lancet piercing movement is modulated by an adjustable thrust and/or an adjustable braking.

6. The lancet drive (1) according to the preceding claim, **characterized in that** a magnetic field may be generated by the electromagnetic actuator, which partially compensates, completely compensates, or overcompensates for the retention force.

7. The lancet device (1) according to any one of claims 5 to 6, **characterized in that** the actuator is controllable using the control unit in such a way that the lancet (10) may be accelerated in a retraction movement opposite to the piercing movement after performing a piercing in such a way that the lancet (10) returns into the initial position, in which it is retained by the magnetic retention force in the tensioned position.

8. The lancet device (1) according to any one of the preceding claims, **characterized in that** the lancet drive (2) and the lancet (10), in particular a pushrod (3) carrying the lancet (10), the lancet (10), and the drive means, in particular a drive spring (4), form an oscillating system, which may oscillate along the piercing path.

9. The lancet device (1) according to the preceding claim, **characterized in that** it is implemented in such a way that the oscillation energy contained in the oscillation system when performing a piercing using the lancet (10) is recuperated to the greater part and stored as potential energy by renewed tensioning of the drive means in an initial position of the drive means following the piercing, so that the recuperated kinetic energy is available for performing a following piercing and does not have to be expended again.

10. The lancet device (1) according to any one of claims 8 to 9, **characterized in that** it is implemented in such a way that the oscillation energy contained in the oscillation system upon performing a piercing using the lancet (10) is significantly greater than the energy which is required to perform a piercing in the skin.

11. An analysis device for analyzing a medically significant component of a bodily fluid or a piercing aid for taking a sample of a bodily fluid through a piercing in a body, **characterized in that** the analysis device or the piercing aid comprises a lancet device (1) according to any one of claims 1 to 10.

## Revendications

1. Dispositif de lancette (1), au moyen duquel une lancette (10) est coulissante le long d'un chemin de piqûre pour engendrer une blessure de piqûre dans une surface de la peau, notamment pour obtenir un liquide corporel à des fins de diagnostic, comprenant
un entraînement de lancette (2) qui comprend un moyen d'entraînement pour générer une force d'entraînement pour un mouvement de piqûre de la lancette (10) le long du chemin de piqûre en direction de la surface de la peau,
un moyen de retenue pour générer une force de retenue magnétique destinée à maintenir la lancette (10) dans une position contractée, et
un moyen de déclenchement, au moyen duquel la force de retenue peut être diminuée de manière à ce que la lancette (10) soit accélérée en direction de la surface de la peau sous l'effet de la force d'entraînement générée par le moyen d'entraînement,
**caractérisé en ce que**
l'entraînement de lancette comprend une unité de commande pour régler la profondeur de piqûre de la lancette (10), au moyen de laquelle la diminution de la force de retenue peut être commandée de sorte que la profondeur de piqûre souhaitée de la lancette (10) est obtenue en fonction de l'unité de commande lorsque le mouvement de piqûre est déclenché.

2. Dispositif de lancette (1) selon la revendication 1, **caractérisé en ce que** la lancette (10) est réalisée pour l'amortissement libre dans le sens de piqûre jusqu'à un point d'inversion d'oscillation, de sorte que la profondeur de piqûre de la lancette (10), du moins dans une zone de profondeur de piqûre pratiquement utilisable et réglable par un moyen de l'unité de commande , n'est pas limitée par une butée de profondeur de piqûre mécanique.

3. Dispositif de lancette (1) selon l'une quelconque des revendications précédentes, **caractérisé en ce que** le moyen d'entraînement comprend un ressort d'entraînement (4), le ressort d'entraînement (4) pouvant être maintenu dans un état contracté par la force de retenue magnétique.

4. Dispositif de lancette (1) selon l'une quelconque des revendications précédentes, **caractérisé en ce que** l'entraînement de lancette (2) comprend une plaque d'ancrage qui est disposée sur une tige de poussée (3) portant la lancette (10) et **en ce qu'**elle est réalisée en fer, dans une autre matière ferromagnétique ou en tant qu'aimant permanent.

5. Dispositif de lancette (1) selon l'une quelconque des revendications précédentes, **caractérisé en ce que** l'entraînement de lancette (2) comprend un acteur électromagnétique agissant sur le mouvement de la lancette (10), cet acteur pouvant être commandé par l'unité de commande pour régler la profondeur de piqûre, de manière à ce que le mouvement de piqûre de la lancette soit modulé par une poussée réglable et/ou par un freinage réglable.

6. Dispositif de lancette (1) selon l'une quelconque des revendications précédentes, **caractérisé en ce qu'**un champ magnétique, qui compense en partie la force de retenue, la compense complètement ou la surcompense, peut être généré par l'acteur électromagnétique.

7. Dispositif de lancette (1) selon l'une quelconque des revendications 5 à 6, **caractérisé en ce que** l'acteur peut être commandé au moyen de l'unité de commande de manière à ce que la lancette (10), après la réalisation d'une piqûre, puisse être accélérée dans un mouvement de retour opposé au mouvement de piqûre, de sorte que la lancette (10) revient en position initiale dans laquelle elle est maintenue par la force de retenue magnétique dans la position contractée.

8. Dispositif de lancette (1) selon l'une quelconque des revendications précédentes, **caractérisé en ce que** l'entraînement de lancette (2) et la lancette (10), notamment une tige de poussée (3) portant la lancette (10), la lancette (10) et le moyen d'entraînement, notamment un ressort d'entraînement (4), forment un système oscillant qui peut osciller le long du chemin de piqûre.

9. Dispositif de lancette (1) selon l'une quelconque des revendications précédentes, **caractérisé en ce qu'**il est réalisé de manière à ce que l'énergie d'oscillation contenue dans le système d'oscillation lors de la réalisation d'une piqûre avec la lancette (10) soit de nouveau obtenue en tant qu'énergie potentielle pour une majeure partie par une nouvelle contraction, réalisée après la piqûre, du moyen d'entraînement dans une position initiale du moyen d'entraînement et soit mémorisée, de sorte que l'énergie cinétique de nouveau obtenue soit disponible pour la réalisation d'une piqûre suivante et qu'elle ne doive pas être nouvellement appliquée.

10. Dispositif de lancette (1) selon l'une quelconque des revendications 8 à 9, **caractérisé en ce qu'**il est réalisé de manière à ce que l'énergie d'oscillation contenue dans le système d'oscillation, lors de la réalisation d'une piqûre avec la lancette (10), soit nettement supérieure à l'énergie qui est nécessaire pour réaliser une piqûre dans la peau.

11. Appareil d'analyse destiné à l'analyse d'un composant médical important d'un liquide corporel ou aide de piqûre destinée au prélèvement d'un échantillon d'un liquide corporel par une piqûre dans un corps, **caractérisé en ce que** l'appareil d'analyse resp. l'aide de piqûre comprend un dispositif de lancette (1) selon l'une quelconque des revendications 1 à 10.
